Europäisches Patentamt

**European Patent Office**

**Office européen des brevets**

(11) Publication number: **0 196 290**
A2

# EUROPEAN PATENT APPLICATION

(21) Application number: **86870037.8**

(22) Date of filing: **24.03.86**

(51) Int. Cl.⁴: **C 12 P 21/00,** A 61 K 39/02, G 01 N 33/569

(30) Priority: **25.03.85 US 715528**
**31.07.85 US 761178**

(43) Date of publication of application: **01.10.86**
**Bulletin 86/40**

(84) Designated Contracting States: **AT BE CH DE FR GB IT LI LU NL SE**

(71) Applicant: **WASHINGTON STATE UNIVERSITY RESEARCH FOUNDATION, C. Clement French Administration Building Washington State University, Pullman WA 99163 (US)**

(72) Inventor: **Barbet, Anthony Francis, Route 1, Box 311, Pullman, WA 99163 (US)**
Inventor: **McGuire, Travis Clinton, S.W. 920 Crestview, Pullman, WA 99163 (US)**
Inventor: **Davis, William Charles, N.W. 300 Yates, Pullman, WA 99163 (US)**
Inventor: **Palmer, Guy Hughes, Route 2 Vox 842, Micanopy Florida 32667 (US)**

(74) Representative: **Tasset, Gérard, SMITHKLINE - RIT rue de l'Institut, 89, B-1330 Rixensart (BE)**

(54) **Anaplasma marginale subunit antigen for vaccination and diagnosis.**

(57) Substantially pure antigenic surface proteins of *Anaplasma marginale* have been identified, and are capable of inducing immune responses in ruminants which neutralizes virulent *Anaplasma marginale*. The antigenic surface proteins have one or more components having a molecular weight of about 105,000 daltons, 86,000 daltons, 61,000 daltons, 36,000 daltons, 31,000 daltons, or 15,000 daltons, and can be purified by an immunoaffinity chromatography process comprising the steps of disrupting *Anaplasma marginale* initial bodies by treatment with a detergent, passing the disrupted initial bodies over a chromatography column comprising an insoluble matrix coupled to monoclonal antibodies against a determinant on said antigenic surface protein to selectively bind said antigenic surface protein to said monoclonal antibodies and recovering the bound substantially pure antigenic surface protein from said insoluble matrix. The antigens have further utility in diagnostic tests for anaplasmosis. They can be synthesized by polypeptide procedures or by genetic engineering.

## ANAPLASMA MARGINALE SUBUNIT ANTIGEN
## FOR VACCINATION AND DIAGNOSIS

### FIELD OF THE INVENTION

The present invention relates to proteins which can be used as antigens in a vaccine to immunize ruminants against challenge with Anaplasma marginale or can be used as antigens for diagnosing anaplasmosis

### BACKGROUND OF THE INVENTION

Anaplasmosis is an arthropod borne homeparasitic disease of cattle and other ruminants caused by a rickettsia Anaplasma marginale (A. marginale). The disease occurs worldwide and severely constrains livestock production in tropical and subtropical regions. The rickettsia is transmitted by ticks, biting flies, and blood contaminated fomites to susceptible animals, where it infects erythrocytes. The intraerythrocytic initial body (a single A. marginale organism in the mature infective stage) reproduces by binary fission to form two to eight initial bodies which are subsequently released to infect additional erythrocytes. During acute infection the parasitemia increases geometrically and severe extravascular anemia occurs. Marked weigh loss, abortion, and death can occur during the acute crisis. Animals that recover from the acute infection remain persistently infected and are a reservoir for transmission to susceptible animals.

Current immunoprophylaxis for anaplasmosis includes premunization with a less virulent A. marginale isolate or A. centrale, a less virulent anaplasma species),followed by tetracycline treatment to control severe infection in some animals, and vaccination with a killed whole organism preparation contaminated with host erythrocyte stroma. Premunition is successful in

0196290

- 2 -

controlling severe clinical disease when cattle are challenged with a virulent isolate. However, clinical disease including weight loss, abortion and occasionally death may result from premunizing inoculum. This inoculum may also transmit other hemoparasites (Babesia, Theileria, Trypanosoma) and viruses (bovine leukemia virus) to the host. Challenge of cattle immunized with the killed A. marginale-erythrocyte stroma vaccine results in mild clinical disease and persistent infection. In addition, the presence of erythrocyte stroma in the vaccine has been shown to induce anti-erythrocyte antibodies that when colostrally transferred cause neonatal isoerythrolysis in nursing calves.

## SUMMARY OF THE INVENTION

It is an object of the present invention to overcome some of the known disadvantages of prior art vaccines and diagnostic tests.

This is accomplished by isolating one or more substantially pure antigenic surface proteins from A. marginale which are capable of inducing an immune response in ruminants. One specific antigenic protein which has been isolated is a protein which has a molecular weight of about 105,000 daltons. This particular protein will be referred to hereafter as Am 105. Other antigenic proteins which have been identified to date have molecular weights of about 80,000 daltons. (AM 86), 61,000 daltons (AM 61), 36,000 daltons (Am 36), and 15,000 daltons (AM 15). The antigen might also comprise an active agent of one or more such proteins, or an immunologically similar protein produced by polypeptide synthesis or genetic engineering.

The substantially pure antigenic surface protein is isolated by purifying A. marginale initial bodies, disrupting the purified A. marginale initial bodies by

use of a detergent, passing the disrupted A. marginale initial bodies through an insoluble matrix, such as an affinity chromatography column, coupled to monoclonal antibodies specific to the protein or which recognize one or more epitopes on the protein to adsorb it onto the insoluble matrix, washing the non-adsorbed materials through the affinity chromatography column, and recovering the substantially pure protein from the insoluble matrix.

The monoclonal antibodies are prepared by vaccinating mice with bovine erythrocytes infected with A. marginale, obtaining lymphocytes from the spleen of infected mice, and fusing the lymphocytes with myeloma cells to produce hybridomas which produce monoclonal antibodies to A. marginale antigens. The hybridomas are then initially screened for anti-A. marginale antibody by indirect immunofluorescence on smears of A. marginale infected blood. The hybridomas are then further screened for monoclonal antibody against the protein by immunoprecipitation of the protein which reacts with the monoclonal antibody and subsequent determination of the molecular weight of the precipitated protein by gel electrophoresis.

Additional monoclonal antibody to the selected protein from the desired hybridomas is produced by collection of ascitic fluid from mice bearing the hybridoma and the collected monoclonal antibody is purified by precipitation, dialysis and chromatography. The purified monoclonal antibody is then coupled to an insoluble matrix such as Sepharose to prepare an immunoaffinity matrix. Partially purified disrupted A. marginale initial bodies are then passed through the immunoaffinity matrix and the selected protein is selectively absorbed onto the matrix. The purified protein which has absorbed onto the matrix is then recovered from the matrix.

The degree of purity of the protein achieved in accordance with the present invention is much higher than the purity of the surface antigen in its natural state. As an example, in its natural state AM 105 is believed to be present in an amount of about 0.1 to 1% of the total protein present in the initial bodies. In its natural state, many other impurities such as about 200 other proteins, carbohydrates, red cells, glycoproteins, and nucleic acid are present. However, the AM 105 can be purified to a purity of at least 90 weight percent, preferably at least 95 weight percent and most preferably at least 98 weight percent. The purified AM 105 has a molecular weight of about 105,000 daltons; and is reactive with monoclonal antibodies produced by cell lines ANA 15D2 and ANA 22B1. The Am 105 is essentially free of contaminating proteins, glycoproteins, carbohydrates, red cells, glycoproteins and nucleic acids.

The Am 105 is a polypeptide having a molecular weight of about 105,000 daltons. However, it is believed that an active fragment of this polypeptide may be effective in inducing immunity to A. marginale in cows. The size of the active fragment may be as small as six to twenty or possibly six to ten amino acids.

The Am 105 or an active fragment thereof may be produced by immunoaffinity chromatography as will be described hereinafter, polypeptide synthesis or genetic engineering (DNA cloning with protein fragment expression).

The Am 105 should be present in a single dose of the vaccine in an amount of 1-200 g, preferably 5-100 g, and most preferably 10-25 g. A single injectable dose will usually have a volume of about 1 ml. Therefore, the concentration of purified surface antigen in an injectable vaccine composition will usually be in the range of from about 1 to about 200 g/ml and preferably about 5 to about 100 g/ml and most preferably 10-25 g/ml. The Am 105 will usually be dissolved in an adjuvant such as Freund's incomplete adjuvant.

The vaccine, in addition to containing the substantially pure surface antigen and optionally an adjuvant, may also contain any other pharmaceutically acceptable carrier or diluent. The pharmaceutically acceptable carrier or diluent is a compound, composition or solvent which is preferably a non-toxic sterile liquid.

In order to immunize ruminants, young animals are inoculated with a vaccine comprising the substantially pure antigenic surface, an active fragment thereof, or an immunologically similar protein produced by polypeptide synthesis or genetic engineering is added to a pharmaceutically acceptable carrier or diluent. Preferably, the animals are successively inoculated with a single dose as defined above at one to six week intervals, preferably two to four weeks intervals about two to eight times, preferably three to five times. The substantially pure protein should be present in the vaccine in an amount effective to induce an immune response in the calves to _Anaplasma marginale_ whereby when the animals are subsequently challenged with virulent _A. marginale_, the degree of acute infection is substantially reduced or even prevented. Injection will usually be performed intramuscularly (i.m.) or subcutaneously (s.c.).

The isolated protein, an active fragment thereof, or an immunologically similar protein produced by polypeptide synthesis or genetic engineering can also be used as the basis of diagnostic tests, such as an Enzyme Linked Immunosorbent Assay for serologic diagnosis of anaplasmosis. When blood samples from suspected animals are tested using such antigens, results distinguishing infected and non-infected animals are obtainable.

DETAILED DESCRIPTION OF THE INVENTION

A.    Preparation of Monoclonal Antibodies to A. marginale.

The source of antigen, mouse immunization protocol, myeloma cell lines used, culture media and conditions, and cell fusion and cloning are described in Davis et al., Development of monoclonal antibodies to Anaplasma marginale and preliminary studies on their application. Proc. Seventh National Anaplasmosis Conf. Oct. 21-23, 1981, Mississippi State University Press. The procedure can be summarized as follows.

Animals - Young Hereford and Holstein cattle were used.

Animals to be infected with A. marginale were splenectomized. Two inbred strains of mice, BALB/c and B10.A(3r), were used as a source of cells to make hybridomas. These and additional strains, B10.A, B10.A(5R) and B10.A(2R), were used as a source of thymocytes for co-culture as feeder cells with hybridoma cells.

Preparation of A. marginale infected erythrocytes - Splenectomized cattle were infected with an Idaho isolate of A. marginale as described in Davis, W.C. et al., Infect. Immun. 22:597-602 (1978). For tissue culture studies, blood was collected in heparin every two to four days after infection. Blood smears were made and stained with Wright's-Giemsa stain and examined for anaplasma bodies. When parasitemia reached 20-50%, blood was collected, centrifuged, freed of the buffy coat and then frozen in 30% dimethylsulfoxide (DMSO) for later use. Additional blood smears were prepared and frozen at -70°C until used to detect monoclonal antibodies.

Preparation of antigen and immunization - Two types of preparations were used as a source of antigen. In the first, frozen cells (50% packed cell volume) with a parasitemic of 30% were thawed and them immediately lysed with Tris (0.01M) ammonium chloride (0.87%) solution (pH 7.2). The lysate was centrifuged at 8000 rpm for 30 minutes; the pellet was washed 3 times in Hanks Balanced Salt Solution (HBSS) and resuspended in 10 ml of HBSS. Six mice [B10.A(3R)] were injected intraperitoneally (i.p.) with 1 ml of lysate. Three days before the spleens were taken for cell fusion, the mice were given an intravenous booster injection of 0.2 ml of lysate. The second preparation, a lysate of infected erythrocytes, was purified on a Renografin density gradient (25-55%) as described in Davis, N.C. et al., Infec. Immun. 22:597-602 (1978). The bands containing anaplasma bodies and contaminating erythrocyte stroma were collected and washed, as described above, then resuspended in 10 ml of HBSS. Five BALB/c mice were injected intraperitoneally with 1 ml for primary immunization and intravenously with 0.2 ml for booster immunization as described. All mice were immunized at least three weeks before receiving a booster injection.

Myeloma cell lines used to produce hybridomas - Several HAT (hypoxanthine, aminopterin and thymidine)- sensitive, tissue-culture-adapted mouse myeloma cell lines were used as fusion partners in the production of antibody producing hybridomas. NS1, a cell line that produces, but does not secrete K light chains, Oi et al., V.T., In Selected Methods in Cellular Immunology (Eds.) B.B. Mishell and SM Shiigi, WH Freeman and Co., pp. 351-372 (1980) and SP2/O-Ag14, a cell line derived from a NS1-BALB/c hybrid that synthesizes neither light or heavy chains, Schulman, M. et al., Nature, 276:279 (1978) were obtained from the Salk Institute. X63-Ag8.653, another cell line that does not produce light or heavy chains, Kearney, J.F. et al., J. Immunol 123: 1548-1550 (1979) was provided by M. Lostrum from the Fred Hutchinson Cancer Research Center, Seattle, WA.

Culture media and culture conditions - Dulbecco's Modified Eagle Medium (DMEM), containing 13% fetal calf serum (FCS), 5 x $10^{-5}$M 2-mercaptoethanol (2ME), penicillin (100 units/ml) and streptomycin (100 mg/ml), was the primary culture medium for maintaining the myeloma cell lines and new hybridoma cell lines. Glutamine (2 mM) was added when the medium was used 14 days after preparation. Fusion and transfer media were prepared by adding HAT and HT (hypoxanthine and thymidine), respectively, according to the method of Littlefield, Monoclonal Antibodies-Hybridomas: A New Dimension of Biological Analysis (Eds.) Kennett, R.H. et al., Plenum Press, pp. 363-416 (1981). RPMI-1640 containing 15% FCS and penicillin/streptomycin was used to culture anaplasma infected erythrocytes, Davis W.C. et al., Infect. Immun. 22:597-602 (1978). All cultures were maintained in a 5% $CO_2$ gas-air mixture at 37°C.

Cell fusion and cloning - Methods for fusing spleen and myeloma cells were similar to those described by Oi et al., supra. Myeloma cells were maintained in log phase of growth in T75 flasks by removing excess cells and feeding every 3 to 4 days. The day before fusing, cells were collected, washed by centrifugation and plated in T75 flasks at $1 \times 10^7$ cells per flask. On the day of use, cells were collected, centrifuged and resuspended in DMEM without FCS. Mice are immunized by injecting disrupted bovine erythrocytes which contain A. marginale initial bodies. Spleen cells were obtained from the freshly killed, immunized mice by injecting the spleen with DMEM, gently tearing it apart with tweezers and then pressing it through a 100 mesh screen into a 50 ml test tube. Following removal of particulate debris, the cells were centrifuged into a pellet and the medium removed. Contaminating mouse erythrocytes were selectively lysed by brief exposure to distilled water (2 ml) and the spleen cells were then quickly diluted in DMEM. Thymocytes to be used as feeder were collected as above (but without water lysis) and suspended in DMEM-FCS-HAT at $1 \times 10^7$ cells per ml. Myeloma cells and spleen cells were counted, then mixed either at a ratio of 5 or 2.5 spleen cells to 1 myeloma cell. Usually, $10^8$ spleen cells were mixed with 2 or $4 \times 10^7$ myeloma cells. The cell mixture was sedimented and the supernatant removed. One ml of a 50% solution of polyethylene glycol (PEG 1540, Baker Chemical Co.) was then placed over the cell pellet and slowly mixed with the cells so as to form a slurry of small ($1 \text{ mm}^3$) clumps of cells. Following 3 minutes of mixing, the cells were slowly diluted by adding 10 ml DMEM in 10 minutes and 20 ml over the next 5 minutes. The resultant mixture of fused cells was centrifuged into a pellet and the cells were resuspended in DMEM-FCS-HAT. Thymocytes were then added to give a mixture containing $10^8$ spleen cells, tumor cells and $1 \times 10^9$ thymocytes. Cells were distributed in ten 96-well culture plates and placed in the incubator. One half the tissue culture medium was replaced every 3 to 4 days. When clones of hybridomas were 300 - 1000 cells in size (usually by 12-18 days), the supernatants were collected and tested by indirect immunofluorescence on smears of infected erythrocytes. Cells from positive wells were identified and transferred to 24-well (2 ml) culture plates. Three to 5 x $10^6$ thymocytes were added as feeder cells to support growth. At 14 days or when the cultures needed to be thinned, the medium was replaced with DMEM-FCS-HT. The cells were maintained in static cultures (1 week in DMEM-FCS-HT then on DMEM-FCS) for two weeks by removing excess cells and feeding every 2-4 days, depending on the rate of replication of individual clones. At this time, a duplicate plate was prepared and allowed to overgrow. The supernatants from this plate were tested for antibody activity. All cell lines identified as antibody producers by this procedure were then taken from the master plate and expanded into 6 well plates (5 ml capacity in each well) as single cultures, 3 wells per cell line. Cells were collected twice and frozen (3-10 x $10^6$ cells in 10% DMSO) in liquid nitrogen. The remaining cells were allowed to proliferate and die. The supernatants were then collected (approximately 15 ml) and frozen for subsequent analysis.

When cell lines producing antibody of immediate interest were identified, they were taken from the freezer, thawed and cloned by limiting dilution immediately or following 24-hrs. culture. Usually, hybridoma cells were plated in 2 to 6 96-well plates, 3 cells per well in the presence of $10^6$ thymocytes. Wells containing single colonies were identified microscopically and supernatants were collected and tested for antibody activity. Cells from positive wells were transferred as above to 24-well plates and then to 6-well plates for colony expansion and preservation. Four to 6 cloned lines were preserved for each line.

In preliminary studies several methods of preparing hybridomas were compared to determine the optimal conditions for use of monoclonal antibody technology in the study of A. marginale. The results obtained revealed that SP2/O-AG 14 and X63-Ag8.653 myeloma cells were the most useful fusion partners because of their growth characteristics, the efficiency of outgrowth of hybridomas, and their inability to secrete light or heavy chains. The fusion ratios found to be the most effective were 5 to 1 for SP2/O-Ag14 and 2.5 to 1 for X63-Ag8.653. Under the culture conditions employed, it was essential to have 2-ME in the culture medium and thymocytes as feeder cells. When these measures were disregarded, outgrowth of the hybridomas was unpredictable; however, when the measures described were taken, 600 to 1000 hybrids were obtained per fusion.

The use of erythrocyte-contaminated preparations of A. marginale as antigen presented no problems. Both the crude lysate and renografin-purified preparations were highly immunogenic. The majority of the hybrids detected in primary culture produced antibody to A. marginale. More anti-erythrocyte antibody producing hybrids were seen when the lysate was used as a source of antigen, however.

When a sufficient number of hybridomas were collected and preserved, they were tested by immunodiffusion to determine the class of antibody being produced. In addition, they were tested by indirect immunofluorescence on smears of infected erythrocytes to determine their patterns of binding to A. marginale or erythrocytes, on cultures of infected monocytes grown in short term culture and on acetone-fixed sections of tissue taken from infected cattle.

B.  Selection of Cell Lines Producing Anti-Am 105 Monoclonal Antibodies.

The hybridoma cell supernatants are initially screened for anti-A. marginale antibody by indirect immunofluorescence on acetone-fixed smears of A. margi- nale infected blood. The positive (antibody producing) cell lines are then further screened for specific production of anti-Am 105 antibodies using immunoprecipi- tation of either [$^{35}$S] methionine radiolabeled or [$^{125}$I] surface radiolabeled A. marginale. The exact procedure for this, which is a slight modification of the procedure reported by Palmer, G.H. et al., J. Immunology, 133:1010 (1984), is as follows.

The immunoprecipitation of surface-radioiodinated initial bodies and erythrocyte stroma was performed by using a modification of the technique described by Sha- piro, S.Z. et al., J. Immunol. Methods, 13:153 (1976). The initial bodies or erythrocytes were disrupted with 1% (v/v) Nonident P-40 and 0.1% (w/v) SDS at 4°C for 30 min, centrifuged at 135,000 x G for 60 min, passed through a 0.2 m filter (Millipore Corp., Bedford, MA), and sonicated for 15 sec at 50 W. Two hundred thousand trichloroacetic acid precipitable cpm were added to 50 ml hybridoma supernatant followed by 10 ml rabbit anti-mouse immunoglobulin in siliconized tubes and were incubated at 4°C for 30 min. One hundred microliters of 10% (v/v) protein A-bearing Staphylococcus aureus (Calbiochem-Behring Corp., La Jolla, CA) were added to each tube, incubated for 30 min at 4°C, and washed six times with Ten buffer (20 mM Tris-HCl, 5 mM EDTA, 0.1 M NaCl, 15 mM NaN$_3$, pH 7.6) containing 0.1% Nonident P-40, and for the first four washes 2 M NaCl, by using centrifugation at 1250 X G. The precipitated label was eluted by boiling the staphylococci-bound complexes for 3 min in 50 ml SDS-PAGE buffer and centrifuging at 1000 x G.

The immunoprecipitates (2000 to 10,000 cpm) and the radioiodinated initial bodies and erythrocytes (200,000 cpm) were electrophoresed on 7.5 to 17.5% (w/v) gradient polyacrylamide gels. The gels were fixed in glassdistilled water:methanol:acetic acid (6:4:1), were dried, and were exposed to Kodak X-Omat AR film (Eastman-Kodak, Rochester, NY) at room temperature for 48 hr to identify the radioiodinated initial body and erythrocyte polypeptides, and at -70°C by using Cronex Quanta III intensifying screens (DuPong, Wilmington, DE) for 72 hr to identify the immunoprecipitates. $^{14}$C-Labeled proteins used for m.w. comparison (Amersham, Arlington Heights, IL) consisted of myosin, 200,000 m.w.; phosphorylase b, 92,500; bovine serum albumin, 69,000; ovalbumin, 46,000; carbonic anhydrase, 30,000; and lysozyme, 14,300. If the hybridoma supernatant contained monoclonal antibodies to Am 105, a band on the X-ray film (autoradiograph) is observed at a molecular weight of about 105,000.

Two cell lines (ANA 15D2 and ANA 22B1) were identified that produced anti-Am 105 antibodies. The cell lines were double cloned and the supernatants concentrated to 0.1 mg immunoglobulin/ml following determination of isotype (both were IgG3). The concentrated supernatants were used for all further testing. The immunoprecipitation of $^{125}$I-Am 105 was repeated with the double cloned hybridoma supernatants. The evidence that Am 105 is an initial body protein and not of erythrocyte origin includes unreactivity of ANA 15D2, ANA 22B1, or rabbit anti-Am 105 antibodies (all positive on initial bodies in infected erythrocytes) with uninfected erythrocytes or infected erythrocyte membranes using immunofluorescence, and failure of these antibodies to immunoprecipitate $^{125}$I radiolabelled erythrocyte ghosts. Additionally, ANA 15D2 and ANA 22B1 immunoprecipitate Am 105 metabolically radiolabelled _in_ _vitro_ during short term erythrocyte culture. It has been demonstrated that during short term cultures $^{35}$S incorporation occurs exclusively in initial bodies. Am 105 is immunoprecipitated as a doublet band seen most clearly with $^{35}$S labelled Am 105 or silver stained Am 105. The doublet is consistently present and most likely represents differences in post translational modification.

In order to test for initial body neutralization using the two anti-Am 105 monoclonal antibodies, $10^{10}$ initial bodies were incubated with pooled ANA 15D2 and ANA 22B1 ascitic fluid, and the mixture was injected into splenectomized calves. Although complete neutralization was not observed, a significant prolongation of the prepatent period occurred (Table 1). The experiment was repeated with the assumption that the prolonged prepatent period observed with $10^{10}$ initial bodies indicated partial neutralization and that complete neutralization would likely occur at a lower infective dose. In the second experiment complete neutralization of initial body infectivity occurred using $10^7$ initlial bodies and partial neutralization as judged by prolonged prepatent periods occurred using $10^8$, $10^9$, and $10^{10}$ initial bodies.

The mechanism of neutralization by the anti-Am 105 monoclonal antibodies is unknown at present. Certainly monoclonal antibodies directed against an initial body determinant necessary for erythrocyte receptor binding would neutralize infectivity. ANA 15D2 and ANA 22B1

may recognize the same or overlapping determinants as they reciprocally inhibit binding of each other to $^{125}$I-Am 105 in a competition radioimmunoassay. Other modes of neutralization include agglutination and when possible across murine-bovine species lines, interaction with bovine effector cells and complement-fixation with initial body lysis.

The effective use of AM 105 as a protective immunogen to prevent bovine anaplasmosis would require that the determinants recognized by the neutralizing monoclonal antibodies be common to all isolates in a given region. Both similarities and differences in protein and antigenic composition among various isolates of A. marginale have been demonstrated. Six isolates from widely geographically separated areas of the U.S. (Florida; Okanogan, Washington; South Idaho; North Texas; Clarkston, Washington; Virginia) have been examined for the presence of determinants recognized by ANA 15D2 and ANA 22B1 using indirect immunofluorescence on acetone fixed blood smears. The determinants were present on 100% of the initial bodies (as determined by comparison with Wright's stained initial bodies in an adjacent section of the smear) in all six isolates. Additionally, the determinants were present at all stages of a primary, acute infection from 1% parasitemia through peak parasitemia with hemolytic crisis. The presence of these determinants now identified as protective antigens on multiple isolates and their presence at all stages of infection fulfill important criteria for use of Am 105 or its fragments as a vaccine. Am 105 and Am 105 polypeptides containing determinants recognized by the neutralizing monoclonal antibodies have been tested as effective immunoprophylaxis for bovine anaplasmosis.

A.   Partial neutralization of infectivity of $10^{10}$ <u>A.</u> <u>marginale</u> initial bodies of monoclonal antibodies (ANA 15D2/22B1).

BALB/c X B10A (3r) mice were injected intraperitoneally (i.p.) with 1.0 ml Pristane and one week later with 2-3 X $10^6$ double cloned hybridoma cells suspected of producing anti-Am 105 antibodies.  Ten days after injection with the hybridoma cells, ascitic fluid was withdrawn from the mice, centrifuged to pellet debris, and passed over a glass wool column.  The indirect fluorescent antibody (IFA) titer is determined.  A strong titer would be 1:16,000.  $10^{10}$ initial bodies are purified from <u>A. marginale</u> (Florida isolate) infec- ted erythrocytes as described in Palmer, G.H. et al., J. Immunology, <u>133</u>:1010 (1984) and resuspended in 2.5 ml RPMI 1640 (2mM L-Glutamine, 25mM HEPES).  The initial bodies are added to 2.5 ml of ascitic fluid.  The initial body-ascitic fluid suspension is briefly vor- texed, incubated for 45 min. at room temperature, and injected into the left semitendinosus muscle of each calf.  Daily blood samples are collected for 75 days post-inoculation (DPI) to determine packed cell volume (PCV) and parasitemia (1000 erythrocytes counted). Probability values (p) are calculated using the pooled t-test; p values of less than 0.05 are considered significant.  NS, not significant.  ND, significance not determined.  The results of a test using two cell lines designated ANA 15D2 and ANA 22B1 which produce monoclonal antibodies to Am 105 and a cell line TRYP 1E1 which produced monoclonal anti-bodies against <u>Try-</u> <u>panosoma brucei</u> are reported in Table I.

## TABLE I

| Parameter | ANA 15D2/22B1 | TRYP 1E1 | Significance |
|---|---|---|---|
| No. infected/ challenged | 6/6 | 4/4 | |
| Mean DPI to 1% parasitemia (range) | 31 (29-37) | 24 (23-25) | $p \leq 0.01$ |
| Mean peak para- sitemia (%) (range) | 51 (30-58) | 71 (69-71) | $p \leq 0.01$ |
| Mean low PCV (range) | 15 (13-17) | 11 (10-11) | NS |
| No. dead/ challenged | 0/6 | 3/4 | ND |

DPI means days post inoculation.
PCV means packed cell volume.

These results indicate that by using $10^{10}$ initial bodies partial neutralization was observed as judged by a significant prolongation of the prepatent period.

B.    Neutralization of infectivity of graded numbers of initial bodies by monoclonal antibodies (ANA 15D2/ 22B1)

0196290

The experiment was repeated with the hypothesis that the prolonged prepatent period observed with $10^{10}$ initial bodies indicated a partial neutralization and that complete neutralization would likely occur at a lower infective dose.

The protocol used was similar to that described in Part a. A constant amount of ascitic fluid was inoculated with either $10^{10}$, $10^9$, $10^8$, or $10^7$ purified A. marginale (Florida) isolate initial bodies, injected into calves, and infection monitored as previously described. The results are reported in Table II.

## TABLE II

| Parameter | No. initial bodies | ANA 15D2/ 22B1 | TRYP 1E1 | Significance |
|---|---|---|---|---|
| No. infected/ | $10^{10}$ | 3/4 | 3/3 | ND |
| challenged | $10^9$ | 4/4 | 3/3 | ND |
| | $10^8$ | 4/4 | 3/3 | ND |
| | $10^7$ | 0/4 | 3/3 | ND |
| Mean DPI to | $10^{10}$ | 33(32-33) | 26(25-28) | $p \leq 0.01$ |
| 1% parasitemia | $10^9$ | .35(33-39) | 28)27-29) | $p \leq 0.01$ |
| (range) | $10^8$ | 37(36-38) | 30(26-32) | $p \leq 0.01$ |
| | $10^7$ | neg @ 75 | 34(34-36) | ND |

These results indicate that complete neutralization of infectivity occurred using $10^7$ initial bodies and partial neutralization, as judged by prolonged prepatent periods, occurred using $10^8$, $10^9$ and $10^{10}$ initial bodies.

C.   Purification of Monoclonal Antibodies.

The steps for purifying the monoclonal antibodies to couple to the immunoadsorbent column are:

1. BALB/c X B10 A (3r) mice were injected intraperitoneally with 1.0 ml Pristane (Aldrich Chemical Co., Milwaukee, WI) and one week later with 2-3 X $10^6$ double cloned hybridoma cells. Ten days later, ascitic fluid was withdrawn from the mice, centrifuged at 1,675 X G to pellet insoluble debris and passed over a glass wool column.

2. A 50% (v/v) $NH_4SO_4$ precipitation is conducted on the wool column effluent and following resuspension in .032M Tris is dialyzed for 24 hrs. against .032 M Tris. This dialyzed sample is chromatographed on a DE-52 cellulose column and eluted using a 0-0.2M NaCl continuous gradient in .032M Tris. The eluted fractions are titered using indirect immunofluorescence on acetone-fixed smears of <u>A. marginale</u> infected blood. The purity of the antibody is determined using sodium dodecyl sulfate-polyacrylamide gel electrophoresis (SDS-PAGE) with detection of protein using Coomassie Blue staining.

D. Preparation of the Monoclonal Immunoaffinity Chromatography Column.

1. The purified monoclonal antibody, ANA 15D2 (pure by Coomassie Blue staining on SDS-PAGE) is dialized against 0.1M $NaHCO_3$, 0.5 M NaCl pH 8.3. The dialyzed protein is coupled to CNBr-activated Sepharose 4B (Pharmacia Fine Chemicals, Piscataway, N.J.) at 10 mg protein/ml settled Sepharose 4B overnight at 4°C rotation. The coupled immunoaffinity matrix (ANA 15D2-Seph 4B) is poured into an Econo column 11.5 ml column (Bio-Rad Lab., Richmond, CA) and stored at 4°C until use.

E.    Source of the _A. marginale_ Initial Bodies.

_A. marginale_ has never been successfully grown in longterm _in vitro_ culture. The source of _A. marginale_ therefore is blood of infected, splenectomized calves. The calves are inoculated with $10^{10}$ initial bodies intramuscularly and then checked daily for evidence of parasitemia using Wright's stained blood smears. When the percentage of infected erythrocytes reaches 40-95%, 4-7 liters of blood are collected into 4 units/ml heparin sulfate. The erythrocytes are washed 3X with phosphate buffered saline, pH 7.2 and the resuspended 1:1 in 31.2% dimethylsulfoxide in phosphate buffered saline. This suspension is frozen in liquid nitrogen and constitutes the _A. marginale_ initial body source.

F.    Disruption and Treatment of _A. marginale_ Initial
        Bodies.

The _A. marginale_ infected erythrocytes ($10^{12}$ will yield approx. 1.0 mg pure Am 105) are thawed from liquid nitrogen at 37°C and washed 5X in phosphate buffered saline using centrifugation at 27,000 X G. The sediment is resuspended in 35 ml phosphate buffered saline, disrupted by 2 min. of sonication at 50 watts (127 X 4 mm titanium probe, Braun-sonic 1510, Braun Instruments, San Francisco, CA) and then washed two times at 1650 X G for 15 min. The purified initial bodies are disrupted in 5 ml 50mM Tris-HCl (pH 8.0, 1.0% Nonident P40, 0.1% sodium dodecyl sulfate, 5mM EDTA, 5mM iodoacetamide, 1mM phenylmethylsulfonylfluoride, 0.1 mM tosyl-L-lysyl chloromethane) and applied to the ANA 15D2-Sepharose 4B monoclonal immunoaffinity column.

G.    Column Chromatography.

The ANA 15D2-Sepharose 4B monoclonal immunoaffinity column is washed with 100 X column volume with TEN buffer (20mM Tris-HCl, 5mM EDTA, 0.1 M NaCl, 25mM NaN$_3$, pH 7.6) 1% NP-40, 0.1% SDS and the approximately 10$^{12}$ disrupted initial bodies are loaded onto the column at a rate of 25 ml/hr.  The unbound proteins are washed out using 100 X column volume TEN with 1.0% NP-40 and 0.1% SDS and then any remaining unbound proteins and the NP-40, SDS (non-dialyzable detergents) are removed by 100 X column volumes TEN without detergent.  The specifically bound Am 105 is eluted using 50mM Tris pH 8.0 with 0.5% deoxycholate and 2M KSCN.

H.    Recovery of Purified Surface Antigen.

The eluted protein (Am 105) is dialyzed against phosphate buffered saline to remove the KSCN and deoxycholate.  Am 105 is quantitated using a modified Lowry protein assay and frozen at -70°C until use.

I.    Preparation of Vaccine.

Am 105 is thawed and suspended in Freund's incomplete adjuvant to produce a vaccine in which substantially pure Am 105 is present in an amount of 10, 25 or 100 mg/ml.

J.    Immunization Studies.

Groups comprised of 5 Holstein calves, weighing approximately 100 kg, were immunized 4 times with 100 mg of either ovalbumin emulsified in Freund's complete adjuvant (group 1), Am 105 emulsified in Freund's incomplete adjuvant (group 2), or Am 36 emulsified in Freund's incomplete adjuvant (group 3).  The immunizations were conducted on day 1, day 17, day 35 and day 59.  Group 4, which consisted of 4 calves, was not immunized. The antibody response of the 4 groups to Am 105 was determined using a radioimmunoassay based on $^{125}$I-Am 105.

The calves were challenged on day 83 with $10^8$ purified Florida isolate A. marginale initial bodies. The calves were monitored for infection by daily clinical examination, determination of hematocrit, and examination of Wright's stained blood smears for presence of parasites.

The results are presented in Table III.

## TABLE III

|  | Group 1 | Group 2 | Group 3 | Group 4 |
|---|---|---|---|---|
| Titer to Am 105 | $10^2$ | $10^4$ | $10^2$ | 0 |
| No. infected/no. challenged | 5/5 | 3/5 | 3/5 | 4/4 |
| x days to infection | 33 | 39 ($p \leq .01$)[a] | 38 | 29 |
| peak parasitemia | 5.4 | <.01 ($p \leq .01$) | 1.5 | 4.2 |
| x low PCV[b] | 24.4 | 31 ($p \leq .01$) | 28 | 23 |

[a] Significance: p values were calculated by the pooled t-test. Probability values of less than 0.05 were considered significant.

[b] PCV = packed cell volume.

The significant elongation of the prepatent period (days until infection was detected), significant reduction in parasitemia, significant difference in PCV, and complete protection in 2 of 5 Am 105 vaccinates relative to calves immunized with ovalbumin indicates that Am 105 is capable of inducing significant protection against challenge with A. marginale.

K.    General Discussion.

The six major A. marginale initial body surface proteins identified to date (Am 105, Am 86, Am 61, Am 36, Am 31, Am 15) each have a surface location on the initial body. Evidence for the surface nature of Am 105, Am 86, Am 61, Am 36, Am 31, and Am 15 proteins was obtained by radioiodination of the proteins on intact initial bodies using a membrane impermeant radiolabeling technique (lactoperoxidase) (Palmer, GH, McGuire TC: J. Immunol. 133:1010-1015, 1984). A. marginale initial bodies were purified from parasitized erythrocytes by using ultrasonic disruption and differential centrifugation. The initial bodies were intact as determined by electron microscopy, were not agglutinated by anti-bovine erythrocyte sera and were infective. Nine initial body proteins were surface radioiodinated using lactoperoxidase (Am 220, Am 105, Am 86, Am 61, Am 56, Am 42, Am 36, Am 31 and Am 25) and five (Am 105, Am 86, Am 61, Am 36 and Am 31) were precipitated by neutralizing antibody. The latter five proteins are major surface proteins and one or more of these proteins alone or in combination might be incorporated in a vaccine or diagnostic test. In fact, the date presented in Table III shows that either purified Am 105 or Am 36 will induce protective immunity against virulent A. marginale challenge in calves. That purified proteins will work as vaccines indicates that similar results might be achieved with synthetic peptides of 6 amino acids or more mimicking the antigenic structure of the biologically active epitopes, with antigens expressed in heterologous bacteria containing the genes coding for the biologically active epitopes of the surface proteins or with one or more antigens expressed in virus vectors containing the genes coding for biologically active epitopes of the surface proteins.

The five major A. marginale initial body surface proteins (Am 105, Am 86, Am 61, Am 36 and Am 31) bear epitopes recognized by neutralizing antibody. Antiserum prepared by immunization of rabbits with purified A. marginale initial bodies completely neutralized the infectivity of $10^{10}$ purified initial bodies for splenectomized cattle. Using the technique of immunoprecipitation these five proteins were recognized by the neutralizing antibody, demonstrating their potential roles, either individually or in combination, in inducing neutralizing antibody and therefore their use as an improved vaccine for cattle. The recognition of these 5 surface proteins was consistent regardless of the isolate (Florida, Washington-O, Virginia) of A. marginale used to immunize the rabbits to prepare the antiserum.

It has been shown that Am 105 and Am 36 each bear an epitope common among all A. marginale isolates tested (Florida, Washington-O, Washington-C, Virginia, N. Texas, S. Idaho, Kansas, Oklahoma, Kapiti (Kenya), and Israel-round and Israel-tails) and to A. centrale (a less virulent species) that are capable of inducing neutralizing antibody. The purification of Am 105 or Am 36 by monoclonal antibody immunoaffinity chromatography and the demonstration of its ability to induce protection in cattle immunized with the protein clearly shows their importance, either alone or in combination with other surface proteins, as an improved vaccine against anaplasmosis. These Am 105 or Am 36 epitopes are completely protease sensitive and do not bear any carbohydrate residues and as such can be easily mimicked by short (minimum 6 amino acids) synthetic peptides or by polypeptides expressed in a foreign bacterium or virus containing the gene coding for the epitopes. The availability of the monoclonal antibody makes both the synthetic peptide and the gene cloning procedures alternative approaches to vaccine development.

The six surface proteins (Am 105, Am 86, Am 61, Am 36, Am 31, Am 15) identified to date are specifically recognized by serum taken from cattle over a period of 30 days to 255 days post-infection. This recognition is consistent regardless of the isolate used to infect the cattle (Florida, Virginia, N. Texas). This specific recognition is required for selection of A. marginale proteins to be used individually or in combination as the antigen in an improved serologic assay to diagnose anaplasmosis in cattle. These supporting data point to use of these proteins for diagnosing anaplasmosis. The isolation and incorporation of these proteins into a serologic assay for diagnosis is a straightforward technical procedure. The findings to date also indicate potential use of a synthetic peptide of 6 amino acids or more or a polypeptide expressed in a vector organism as immunologically equivalent agents for diagnostic purposes.

Am 105 and Am 36, isolated by monoclonal immunoaffinity chromatography and coated into wells of a microtiter plate at 5 to 100 ng/well, have been tested as the basis of an Enzyme Linked Immunosorbent Assay (ELISA) for serologic diagnosis of anaplasmosis. Each assay has been found capable of differentiating non-

infected from <u>A. marginale</u> or <u>A. centrale</u> infected cattle at periods from 30-225 days post-infection and was accurate regardless of the isolate used to infect the cattle (Florida, N. Texas, Virginia, Washington-O, Washington-C, Idaho, Kenya, Israel-round, Israel-tailed). The present serologic assay is based on the isolated whole Am 105 or Am 36. These findings, however, imply the potential use of an immunologically similar synthetic peptide of six amino acids or more or a polypeptide expressed in a vector organism.

Proteins of 105,000 daltons (Am 105), 86,000 daltons (Am 86), 61,000 daltons (Am 61), 31,000 daltons (Am 31) and 15,000 daltons (Am 15), all identified as surface proteins, are strongly antigenic as evidenced by antibody in <u>A. marginale</u>-infected cattle. In addition, dilutions of the post-infection sera have high titers to Am 86 and Am 15 throughout infection, indicating a preferential response. Use of Am 86 alone or in combination with Am 105, Am 61, Am 31, or Am 15 as an antigen in a diagnostic test is implied from these findings. The present stage of this research also points to potential use of an immunologically similar synthetic peptide of six amino acids or more or a polypeptide expressed in a vector organism which has epitopes recognized by post-infection sera as antigens in diagnostic tests for anaplasmosis. Hybridomas ANA 15D2 and ANA 22B1 have been deposited in the American Type Culture Collection, Rockville, Maryland, under the terms of the Budapest Treaty.

CLAIMS FOR THE CONSTRACTING STATES :
BE, CH, DE, FR, GB, IT, LI, LU, NE, SE

1. A substantially pure antigenic surface protein of Anaplasma marginale, an active fragment thereof, or an immunologically similar protein produced by polypeptide synthesis or genetic engineering which, when inoculated into a ruminant, is capable of inducing an immune response is said ruminant to Anaplasma marginale.

2. The substantially pure antigenic surface protein of claim 1, which has a purity of at least 90% by weight.

3. The substantially pure antigenic surface protein of claim 1, which has a purity of at least 95% by weight.

4. The substantially pure antigenic surface protein of claim 1, which has a molecular weight of about 105,000 daltons.

5. The substantially pure antigenic surface protein of claim 1, which has a molecular weight of about 86,000 daltons.

6. The substantially pure antigenic surface protein of claim 1, which has a molecular weight of about 61,000 daltons.

7. The substantially pure antigenic surface protein of claim 1, which has a molecular weight of about 36,000 daltons.

8. The substantially pure antigenic surface protein of claim 1, which has a molecular weight of about 31,000 daltons.

9. The substantially pure antigenic surface protein of claim 1, which has a molecular weight of about 15,000 daltons.

10. The substantially pure antigenic surface protein of claim 1 comprising one or more proteins having molecular weights of about 105,000 daltons, 86,000 daltons, 61,000 daltons, 36,000 daltons, 31,000 daltons and 15,000 daltons.

11. The substantially pure antigenic surface protein of claim 2, prepared by the process comprising the steps of disrupting A. marginale initial bodies by treatment with a detergent, passing the disrupted initial bodies over an insoluble matrix coupled to monoclonal antibodies against a determinant on said antigenic surface protein to selectively bind said antigenic surface protein to said monoclonal antibodies and recovering the bound substantially pure antigenic surface protein from said insoluble matrix.

12. An anaplasmosis vaccine, comprising the substantially pure antigenic surface protein, an active fragment thereof, or an immunologically similar protein produced by polypeptide synthesis or genetic engineering of claim 1, in an amount sufficient to induce the formation of antibodies which neutralize virulent A. marginale in a ruminant; and a pharmaceutically acceptable carrier or diluent.

13. The vaccine of claim 12, wherein said antigenic surface protein is present in an injectable solution in an amount of about 1 to 200 g/ml of solution.

14. The vaccine of claim 12, which further includes Freund's incomplete adjuvant.

15. An anaplasmosis vaccine, comprising the substantially pure antigenic surface protein of claim 11, in an amount sufficient to induce the formation of antibodies which neutralize virulent A. marginale in a ruminant; and a pharmaceutically acceptable carrier or diluent.

16. A process for preparing an antigenic surface protein of Anaplasma marginale which comprises disrupting A. marginale initial bodies and recovering therefrom a protein which is capable of inducing an immune response in the ruminant to A. marginale.

17. The process of claim 16 which comprises the steps of disrupting A. marginale initial bodies by treatment with a detergent; passing the disrupted initial bodies over an insoluble matrix coupled to monoclonal antibodies against a determinant on said antigenic surface protein to selectively bind said antigenic surface protein to said monoclonal antibodies; and recovering the bound substantially pure antigenic surface protein from said insoluble matrix.

18. The process of claim 16 which comprises the steps of disrupting bovine erythrocytes which are infected with Anaplasma marginale initial bodies by sonication; recovering purified Anaplasma marginale initial bodies from the disrupted erythrocytes; disrupting the purified Anaplasma marginale initial bodies by treatment with a detergent; passing the disrupted initial bodies over an insoluble matrix having monoclonal antibodies against a determinant on an antigenic surface protein of Anaplasma marginale bound thereto selectively bind an antigenic surface protein to said monoclonal antibodies; and recovering the bound substantially pure antigenic surface protein from said insoluble matrix.

19. The process of claim 16, 17 or 18 in which the antigenic surface protein has a molecular weight of 105,000 daltons, 86,000 daltons, 61,000 daltons, 36,000 daltons, 31,000 daltons or 15,000 daltons.

20. A serologic diagnostic test for anaplasmosis utilizing the substantially pure antigenic surface protein, active fragment, or protein produced by polypeptide synthesis or genetic engineering of claim 1.

21. A method for diagnostic testing of ruminants to detect the presence of Anaplasma marginale, comprising the steps of drawing a blood sample from an animal, subjecting the blood samples to the diagnostic test of claim 20; and analyzing the results to distinguish non-infected animals from those that are infected.

## CLAIMS FOR THE CONTRACTING STATE AT

1. A process for preparing an antigenic surface protein of Anaplasma marginale which comprises disrupting A. marginale initial bodies and recovering therefrom a protein which is capable of inducing an immune response in the ruminant to A. marginale.

2. The process of claim 1 which comprises the steps of : disrupting A. marginale initial bodies by treatment with a detergent; passing the disrupted initial bodies over an insoluble matrix coupled to monoclonal antibodies against a determinant on said antigenic surface protein to selectively bind said antigenic surface protein to said monoclonal antibodies; and recovering the bound substantially pure antigenic surface protein from said insoluble matrix.

3. The process of claim 1 which comprises the steps of : disrupting bovine erythrocytes which are infected with Anaplasma marginale initial bodies by sonication; recovering purified Anaplasma marginale initial bodies from the disrupted erythrocytes; disrupting the purified Anaplasma marginale initial bodies over an insoluble matrix having monoclonal antibodies against a determinant on an antigenic surface protein of Anaplasma marginale bound thereto to selectively bind an antigenic surface protein to said monoclonal antibodies; and recovering the bound substantially pure antigenic surface protein from said insoluble matrix.

4. The process of claim 1, 2 or 3, in which the antigenic surface protein has a molecular weight of 105,000 daltons, 86,000 daltons, 61,000 daltons, 36,000 daltons, 31,000 daltons or 15,000 daltons.